# EUROPEAN PATENT SPECIFICATION

(11) **EP 1 254 374 B1**
(45) Date of publication and mention of the grant of the patent: **18.04.2007**
(21) Application number: 01912066.6
(22) Date of filing: 08.02.2001
(51) Int. Cl.: G01N 33/68

(54) **METHOD FOR DIAGNOSING EFFICACY OF XENOTYPIC ANTIBODY THERAPY**
VERFAHREN ZUR DIAGNOSE DER EFFEKTIVITÄT DER XENOTYPISCHEN ANTIKÖRPERTHERAPIE
DETERMINATION DE L'EFFICACITE D'UNE THERAPIE PAR ANTICORPS XENOTYPIQUES

(30) Priority: 08.02.2000 US 181008 P; 04.05.2000 US 201868 P
(43) Date of publication of application: 06.11.2002
(73) Proprietor: AltaRex Medical Corporation, Edmonton Alberta T6E 6S4 (CA)
(72) Inventor: NOUJAIM, Antoine, Edmonton, Alberta T6M 2K4 (CA)
(74) Representative: Chapman, Paul Gilmour
(86) International application number: PCT/IB2001/000423
(87) International publication number: WO 2001/059452

(56) References cited:
- SCHULTES BIRGIT C ET AL: "Anti-idiotype induction therapy: Anti-CA125 antibodies (AB3) mediated tumor killing in patients treated with Ovarex mAb B43.13 (Ab1)." CANCER IMMUNOLOGY IMMUNOTHERAPY, vol. 46, no. 4, June 1998 (1998-06), pages 201-212, XP002194278 ISSN: 0340-7004
- MADIYALAKAN R ET AL: "Antiidiotype Induction Therapy: Evidence for the Induction of Immune Response through the Idiotype Network in Patients with Ovarian Cancer after Administration of Anti-CA125 Murine Monoclonal Antibody B43.13." HYBRIDOMA, vol. 14, no. 2, 1995, pages 199-203, XP001064580 ISSN: 0272-457X
- QI W ET AL: "Induction of idiotype network to anti-MUC-1 antibody in breast cancer." PROCEEDINGS OF THE AMERICAN ASSOCIATION FOR CANCER RESEARCH ANNUAL, vol. 39, March 1998 (1998-03), page 367 XP002194279 89th Annual Meeting of the American Association for Cancer Research;New Orleans, Louisiana, USA; March 28-April 1, 1998, March, 1998 ISSN: 0197-016X
- REINARTZ S ET AL: "Evaluation of immunological responses in patients with ovarian cancer treated with the anti-idiotype vaccine ACA125 by determination of intracellular cytokines-A preliminary report." HYBRIDOMA, vol. 18, no. 1, February 1999 (1999-02), pages 41-45, XP001064593 ISSN: 0272-457X
- SCHLEBUSCH HARALD ET AL: "A Monoclonal Antiidiotypic Antibody ACA 125 Mimicking the Tumor-Associated Antigen CA 125 for Immunotherapy of Ovarian Cancer." HYBRIDOMA, vol. 14, no. 2, 1995, pages 167-174, XP001064579 ISSN: 0272-457X
- KOEHLER S ET AL: "Immunotherapy of ovarian carcinoma with the monoclonal anti-idiotype antibody ACA125: Results of the phase Ib study." GEBURTSHILFE UND FRAUENHEILKUNDE, vol. 58, no. 4, April 1998 (1998-04), pages 180-186, XP001064559 ISSN: 0016-5751
- LEVEUGLE B ET AL: "PSA-directed immunotherapy of prostate cancer." PROCEEDINGS OF THE AMERICAN ASSOCIATION FOR CANCER RESEARCH ANNUAL, vol. 39, March 1998 (1998-03), page 355 XP002194280 89th Annual Meeting of the American Association for Cancer Research;New Orleans, Louisiana, USA; March 28-April 1, 1998, March, 1998 ISSN: 0197-016X
- WAGNER U A ET AL: "IMMUNOTHERAPY OF ADVANCED OVARIAN CARCINOMAS BY ACTIVATION OF THE IDIOTYPIC NETWORK" BIOTECHNOLOGY THERAPEUTICS, vol. 3, no. 1-2, 1992, pages 81-89, XP001053690 ISSN: 0898-2848

## Description

### BACKGROUND OF THE INVENTION

### Field of the invention

The invention relates to xenotypic antibody-mediated immunotherapy.

### Summary of the related art

Xenotypic antibody-mediated immunotherapy is an emerging therapeutic approach for a variety of diseases. Ongoing clinical trials utilize murine monoclonal antibodies directed against CA125 antigen to treat ovarian cancer in humans. Ovarian cancer patients in traditional therapies have a high frequency of short-term relapse. Unfortunately, relapse is commonly not detected until the reappearance of CA125 antigen in the patient's blood stream. By that time, medical intervention options may be more limited than they might have been if the relapse could have been predicted earlier.

There is therefore a need for a method for predicting the likelihood of success of xenotypic antibody-mediated immunotherapy.

### BRIEF SUMMARY OF THE INVENTION

The invention provides a method for predicting the likelihood of success of xenotypic antibody-mediated immunotherapy. The invention further provides a method for diagnosing the period of time after xenotypic antibody-mediated immunotherapy during which a patient will be free from relapse.

The invention provides a method for diagnosing the efficacy of xenotypic antibody-mediated immunotherapy, the method comprising measuring the level of T cell stimulation response to the target antigen of the xenotypic antibody produced in response to xenotypic antibody administration. In certain embodiments, the T cell response is a helper T cell response, a cytotoxic T cell response, or a combination of helper and cytotoxic T cell responses.

### BRIEF DESCRIPTION OF THE DRAWINGS

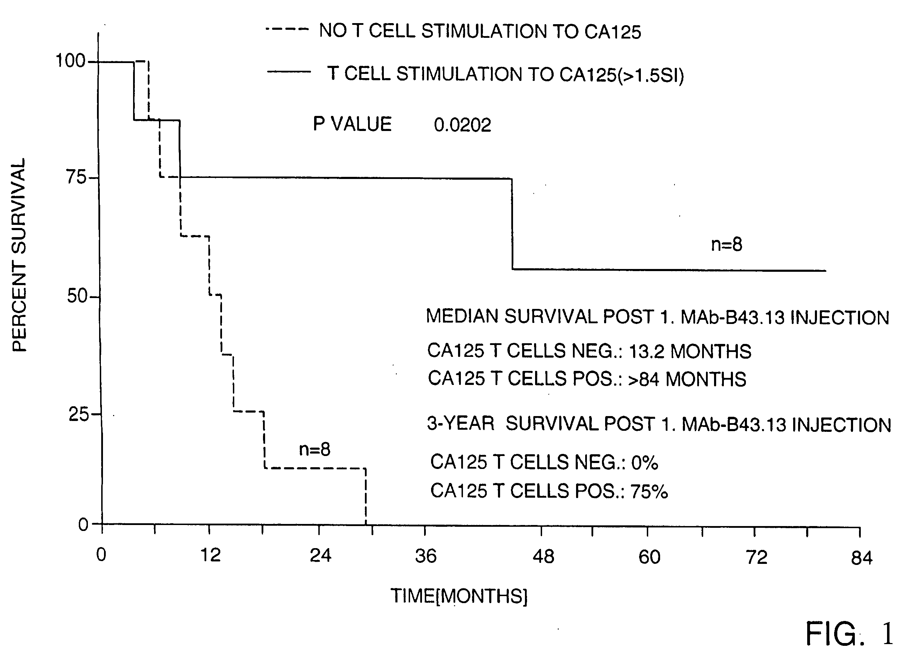

Figure 1 shows correlation between increased level of T cell stimulation and increased survival.

### DETAILED DESCRIPTION OF THE PREFERRED EMBODIMENTS

The invention relates to xenotypic antibody-mediated immunotherapy. The invention provides a method for predicting the likelihood of success of xenotypic antibody-mediated immunotherapy. Accordingly, using the methods of the invention, an assessment may be made as to whether xenotypic antibody-mediated immunotherapy is efficacious and thus should be continued in the patient, or whether the xenotypic antibody-mediated is not efficacious, and the patient should thus be re-evaluated for a possible alternate treatment. The invention further provides a method for diagnosing the period of time after xenotypic antibody-mediated immunotherapy during which a patient will be free from relapse.

As used herein, "diagnosing the efficacy" means predicting the time after administration of a xenotypic antibody at which relapse occurs. By "favorable" diagnosis" is meant a diagnosis that predicts that the time after administration of a xenotypic antibody at which relapse occurs is longer than the time after administration of a placebo (e.g., sugar solution or physiological saline solution) at which relapse occurs. In all of the aspects of the invention, a favorable diagnosis of efficacy increases the time to disease progression or increases the likelihood of survival of the patient.

As used herein, "efficacy" means having the ability to delay disease progression or extend the life of a diseased patient. "Relapse" means the return of clinically observable signs or symptoms of disease. "Xenotypic antibody-mediated immunotherapy" means the administration of an antibody from one species of animal to a second species of animal having a disease, where the antibody forms an antibody-antigen pair with an antigen in the body of the second species that is associated with the disease, thereby reducing or eliminating clinically relevant signs or symptoms of the disease, as may be determined by any ordinarily skilled health care professional (e.g., a nurse or a physician). By "target antigen associated with the disease" is meant an antigen which is found in greater quantities or as a altered protein in patients suffering from a disease. Non-limiting examples of target antigens associated with disease are CA125, which is associated with ovarian cancer, and prostate specific antigen, which is associated with prostate cancer.

"Xenotypic antibody" means antibody from another species. (Note that "antibody" and "antibodies" are used interchangeably throughout). Thus, if the patient is a human, the xenotypic antibodies would be non-human antibodies. As used herein, by "administer" or "administering" or "administration" is meant delivery of a xenotypic antibody by any suitable means, including, without limitation, intramuscular administration, intradermal, intravenous, intra-arterial, peritoneal, subcutaneous, and intra-lymphatic. Those of ordinary skill in the art will realize that a xenotypic antibody can be administered according to the methods of the invention in any physiologically acceptable formulation (e.g., with saline solution). Methods for making pharmaceutically acceptable carriers and formulations thereof are found, for example, in Remington's Pharmaceutical Sciences (18th edition), ed. A. Gennaro (1990) Mack Publishing Company, Easton, PA.

Preferred diseases treated by xenotypic antibody-mediated immunotherapy include cancers, inflammatory diseases, and bacterial, parasitic, and viral infections. Particularly preferred are ovarian cancer, breast cancer and prostate cancer. Preferred xenotypic antibodies include, without limitation, murine monoclonal antibodies. Particularly preferred antibodies include, without limitation, OvaRex^{™} (which specifically binds to the CA125 antigen BrevaRex^{™} (which specifically binds to the MUC-1 antigen) and ProstaRex^{™} (which specifically binds to prostate specific antigen).

Administration of the xenotypic antibody results in a T-cell response against the target antigen of the xenotypic antibody that has a stimulation index of greater than 1.5 times higher than before the administration of the xenotypic antibody. The stimulation index can be determined according to standard T cell stimulation assays (*e.g.*, 1.5 times higher ³H-thymidine uptake by T cells proliferating in the presence of the target antigen as compared to the ³H-thymidine uptake by T cells proliferating in the absence of the target antigen).

The invention provides a method for diagnosing the efficacy of xenotypic antibody-mediated immunotherapy, the method comprising measuring the level of T cell stimulation to the target antigen of the xenotypic antibody produced in response to xenotypic antibody administration. The term "level of T cell stimulation to the target antigen of the xenotypic antibody" means the stimulation index of T cells specific for the target antigen of the xenotypic antibody. T cell stimulation can be determined, for example, by incubating patient cells in vitro with the target antigen (*e.g.*, CA125 antigen) or tissue culture media only (*i.e.*, no antigen), pulsing the cells with ³H-thymidine, and counting the amount of ³H uptake by the cells. In this assay, the stimulation index is a comparison of the amount of ³H taken up by cells in the presence of target antigen versus the amount of ³H taken up by cells in the absence of antigen. Another method for measuring T cell stimulation (*e.g.*, for cytotoxic T cell stimulation) is a ⁵¹Cr release assay, where target cells (*i.e.,* MHC matched) are incubated with the target antigen or tissue culture media only (*i.e.,* no antigen), and then pulsed with ⁵¹Cr. Next, patients' cells are added and the mixture of cells incubated for an amount of time, and then the amount of ⁵¹Cr released by lysed cells is measured. In this assay, the stimulation index is a comparison of the ⁵¹Cr released by cells in the presence of target antigen versus the amount of ⁵¹Cr released by the cells in the absence of antigen.

All other definitions and preferred embodiments are as described for the first aspect of the invention. Preferably, the level of T cell stimulation to the target antigen of the xenotypic antibody is at least 1.5-fold higher than before administration of the xenotypic antibody.

Survival time was compared with the level of T cell stimulation to the target antigen of the xenotypic antibody. The results are shown in Figure 6. Median survival time for patients having at least a 1.5-fold increase in T cell stimulation index was 84 months. Three year survival in these patients was 75%. Median survival time for patients having less than a 1.5-fold increase in T cell stimulation index was 13.2 months. Three year survival in these patients was 0%.

The following example is provided to further illustrate certain preferred embodiments of the invention and is not to be construed as narrowing the scope of the invention.

### Example 1

### Determination of HAMA and T-cell response

A determination of the HAMA response of a patient may be made by using any of the numerous methods for determining an anti-murine responsive antibody concentration known to those of skill in the art of the invention. For example, any standard immunological assay, including, without limitation, ELISA or RIA, may be used to determine the HAMA response of a patient receiving treatment with the murine antibody of the invention. Such standard immunological assays are described, for example, in Ausubel et al. (1999) Current Protocols in Molecular Biology. John Wiley & Sons, New York, NY; and Coligan et al. (1999) Current Protocols in Immunology, John Wiley & Sons, New York, NY.

In one non-limiting example, a group of human patients is administered a murine antibody according to the methods of the invention. At various time points following administration of the murine antibody according to the invention, a blood sample from each patient is collected and measured for the amount of antibody present in the sample that is responsive to a murine antibody, such as the murine antibody that is used for administration. The amount of human antibody reactive to the murine antibody (*i.e.*, the amount of the HAMA response) of each patient may be easily measured.

For example, using an ELISA-based assay to titer the HAMA response, an amount of murine antibody is used to coat the bottom of the wells in a 96 well plate. Limiting dilutions of each patient's blood sample are added to the wells of the plate, and under conditions such that the antibody in the patients' blood can specifically bind to the murine antibody.

Following antibody-specific binding, the plate is rinsed, such that the human antibody that did not specifically bind to the murine antibody coated onto the 96 well plate is removed. Next, a secondary anti-human antibody is added to each plate, and under conditions such that antibody-specific binding may occur. Preferably, the anti-human antibody is labeled with a fluorophore, such that bound secondary antibody can be detected using a 96 well plate reader. The amount of HAMA activity in the patient's blood can be readily determined by determining the binding of of secondary antibody to the 96 well plate.

Whether or not the patients' blood includes Ab3 antibody (*i.e.*, antibody produced by the patient that specifically binds to the target antigen) can be similarly determined by ELISA by determining whether the antibody in the patients' sera binds to a 96 well coated with the target antigen. Secondary anti-human antibody binding to the plate indicates the patients' are able to generate an Ab3 response following administration of a xenotypic antibody that specifically binds to the target antigen.

Whether or not the patients' blood includes T cells (helper and/or cytotoxic) that specifically bind to the target antigen in context of matched MHC can be readily determined by a helper T cell assay (*e.g.*, ³H thymidine uptake assay) or a cytotoxic T cell assay (*e.g.,* a ⁵¹Cr release assay) using MHC matched target cells (*e.g.,* from the patient him/herself) incubated with the target antigen or no antigen (negative control). Any increased proliferation by helper T cells or increased lysis by cytotoxic T cells in the presence of target antigen as compared to no antigen is indicative that the patient has a helper and/or cytotoxic T cell response.

## Claims

1. A method of determining whether to continue to provide xenotypic antibody-mediated immunotherapy to a patient who has a disease associated with a target antigen to which the xenotypic antibody binds comprising measuring *in vitro* the level of a T cell response produced to said target antigen by said patient before and after administration of the xenotypic antibody to the patient, wherein an increase of at least 1.5 fold of a T cell response produced to such antigen after administration of the xenotypic antibody to patient relative to the level of the level of the T cell response produced by the patient prior to the administration of the xenotypic antibody identifies said patient as a target for continuing said xenotypic antibody-mediated immunotherapy.

2. The method of claim 1, wherein the T cell response is a T helper response.

3. The method of claim 2, wherein the T helper response is a cytotoxic T cell response.

4. The method of claim 1, wherein the patient is human.

5. The method of claim 1, wherein the target antigen is selected from CA125, MUC-1, and prostate specific antigen.

6. The method of claim 1, wherein the xenotypic antibody is a murine antibody.

7. The method of claim 6, wherein the murine antibody is a monoclonal antibody.

## Patentansprüche

1. Verfahren zum Bestimmen, ob die Bereitstellung von durch xenotypischen Antikörper vermittelter Immunotherapie an einen Patienten, der eine Krankheit, verbunden mit einem Ziel-Antigen, an welches der xenotypische Antikörper bindet, hat, fortzusetzen ist, umfassend *in vitro* Messen des Niveaus einer T-Zellen-Antwort, erzeugt für das Ziel-Antigen durch den Patienten vor und nach Verabreichung des xenotypischen Antikörpers an den Patienten, wobei eine Zunahme von mindestens dem 1,5-fachen einer T-Zellen-Antwort, erzeugt für derartiges Antigen nach Verabreichung des xenotypischen Antikörpers an den Patienten, relativ zu dem Niveau des Niveaus der T-Zellen-Antwort, erzeugt durch den Patienten vor der Verabreichung des xenotypischen Antikörpers, den Patienten als Ziel zum Fortsetzen der durch xenotypischen Antikörper vermittelten Immunotherapie identifiziert.

2. Verfahren nach Anspruch 1, wobei die T-Zellen-Antwort eine T-Helfer-Antwort ist.

3. Verfahren nach Anspruch 2, wobei die T-Helfer-Antwort eine zytotoxische T-Zellen-Antwort ist.

4. Verfahren nach Anspruch 1, wobei der Patient ein Mensch ist.

5. Verfahren nach Anspruch 1, wobei das Ziel-Antigen aus CA125, MUC-1 und Prostata-spezifischem Antigen ausgewählt ist.

6. Verfahren nach Anspruch 1, wobei der xenotypische Antikörper ein muriner Antikörper ist.

7. Verfahren nach Anspruch 6, wobei der murine Antikörper ein monoklonaler Antikörper ist.

## Revendications

1. Méthode pour déterminer si continuer à dispenser une immunothérapie par la médiation d'un anticorps xénotypique à un patient qui a une maladie associée à un antigène cible auquel l'anticorps xénotypique se lie, comprenant mesurer *in vivo* le niveau d'une réponse des cellules T produite audit antigène cible par ledit patient avant et après l'administration de l'anticorps xénotypique au patient, où une augmentation d'au moins 1,5 fois dans la réponse des cellules T à un tel antigène après l'administration de l'anticorps xénotypique au patient, par rapport au niveau du niveau de la réponse des cellules T produite par le patient préalablement à l'administration de l'anticorps xénotypique, identifie le patient en tant que cible pour continuer ladite immunothérapie par la médiation d'un anticorps xénotypique.

2. La méthode de la revendication 1, dans laquelle la réponse des cellules T est une réponse des cellules T auxiliaires.

3. La méthode de la revendication 2, dans laquelle la réponse des cellules T auxiliaires est une réponse des cellules T cytotoxiques.

4. La méthode de la revendication 1, dans laquelle le patient est humain.

5. La méthode de la revendication 1, dans laquelle l'antigène cible est sélectionné parmi le CA125, le MLTC-1 et l'antigène spécifique de la prostate.

6. La méthode de la revendication 1, dans laquelle l'anticorps xénotypique est un anticorps murin.

7. La méthode de la revendication 6, dans laquelle l'anticorps murin est un anticorps monoclonal.
